# FASCICULE DE BREVET EUROPEEN

(11) **EP 3 823 947 B1**
(45) Date de publication et mention de la délivrance du brevet: **24.08.2022**
(21) Numéro de dépôt: 19753416.7
(22) Date de dépôt: 16.07.2019
(51) Int. Cl.: C07C 17/20, C07C 21/18

(54) **PROCÉDÉ DE PRODUCTION DU TRANS-1-CHLORO-3,3,3-TRIFLUOROPROPÈNE**
VERFAHREN ZUR HERSTELLUNG VON TRANS-1-CHLOR-3,3,3-TRIFLUORPROPEN
PROCESS FOR THE PRODUCTION OF TRANS-1-CHLORO-3,3,3-TRIFLUOROPROPENE

(30) Priorité: 18.07.2018 FR 1856644
(43) Date de publication de la demande: 26.05.2021
(73) Titulaire: ARKEMA FRANCE, 92700 Colombes (FR)
(72) Inventeur: COLLIER, Bertrand, 69491 PIERRE-BENITE CEDEX (FR); PIGAMO, Anne, 69491 PIERRE-BENITE CEDEX (FR); BRUSADELLI, Nicolas, 69491 PIERRE-BENITE CEDEX (FR)
(74) Mandataire: Arkema Patent
(86) Numéro de dépôt international: PCT/FR2019/051776
(87) Numéro de publication internationale: WO 2020/016515

(56) Documents cités:
- US-A- 5 877 359
- US-A1- 2015 197 467

## Description

### Domaine technique de l'invention

La présente invention concerne la production d'hydrochlorofluorooléfines. En particulier, la présente invention se rapporte à la production de 1-chloro-3,3,3-trifluoropropène.

### Arrière-plan technologique de l'invention

Le 3,3,3-trifluoro-1-chloropropène ou encore 1-chloro-3,3,3-trifluoropropène (HCFO-1233zd) existe sous forme de deux isomères : l'isomère cis, à savoir le Z-3,3,3-trifluoro-1-chloropropène (HCFO-1233zdZ), et l'isomère trans, à savoir le E-3,3,3-trifluoro-1-chloropropène (HCFO-1233zdE). Ils ont des points d'ébullition différents, respectivement de 18,5°C pour le composé trans et de 39,5°C pour le composé cis.

Les fluides à base de E-3,3,3-trifluoro-1-chloropropène (HCFO-1233zdE) ont trouvé de nombreuses applications dans des domaines industriels variés, notamment en tant que fluides de transfert de chaleur, propulseurs, agents moussants, agents gonflants, diélectriques gazeux, milieux de polymérisation ou monomères, fluides supports, agents abrasifs, agents de séchage et fluides pour unité de production d'énergie.

La fabrication du HCFO-1233zdE s'accompagne d'une multitude de sous-produits, ayant un point d'ébullition proche du HCFO-1233zdE. Cela conduit à des étapes de purification assez complexes et coûteuses. Les difficultés rencontrées au cours de la purification du HCFO-1233zdE impliquent généralement une perte conséquente en produit recherché. De plus, les sous-produits peuvent former des compositions azéotropiques avec le HCFO-1233zdE, rendant très difficile voire impossible la séparation par distillation simple.

On connait par US 5,877,359 un procédé de préparation du HCFO-1233zdE à partir du 1,1,3,3-tétrachloropropène en phase liquide et en l'absence de catalyseur. On connait également par US 9,643,903 un procédé de fluoration du 1,1,3,3-tétrachloropropène en phase liquide et en l'absence de catalyseur dans un milieu riche en HF. On connait également par US 9,255,045 un procédé de fluoration du 1,1,3,3-tétrachloropropène en 1-chloro-3,3,3-trifluoropropène.

Généralement, la réaction de fluoration est mise en œuvre à une température nécessitant de chauffer le réacteur à une température nettement supérieure à la température de réaction visée. La température élevée des parois du réacteur génère localement une augmentation de la production de sous-produits tels que le cis-1-chloro-3,3,3-trifluoropropène ou la production de produits surfluorés tels que le 1,1,1,3,3-pentafluoropropane ou le 1,3,3,3-tétrafluoropropène.

Il existe un besoin pour un procédé efficace de production du trans-1-chloro-3,3,3-trifluoropropène minimisant la production de sous-produits ou de produits surfluorés.

### Résumé de l'invention

Le demandeur a constaté de manière surprenante que le préchauffage de la matière première de départ, en particulier le préchauffage de l'acide fluorhydrique, à une température nettement supérieure à la température de réaction permettait de limiter le chauffage du réacteur.

La présente invention fournit un procédé de production du trans-1-chloro-3,3,3-trifluoropropène comprenant les étapes de :
i) fourniture d'un réacteur comprenant un couvercle, un fond, des parois latérales connectant ledit fond et ledit couvercle, au moins une ligne d'alimentation en réactifs et au moins une ligne de soutirage des produits formés, ledit réacteur contenant en outre une phase liquide A ;
ii) fourniture d'un courant **B** comprenant de l'acide fluorhydrique chauffé à une température **T1** comprise de 100°C à 170°C et fourniture d'un courant **C** comprenant le 1,1,3,3-tétrachloropropène et/ou 1,3,3,3-tétrachloropropène ; ledit courant **B** et ledit courant **C** alimentant ledit réacteur par l'intermédiaire de ladite au moins une ligne d'alimentation en réactifs,
iii) réaction dans ladite phase liquide A dudit courant **B** avec ledit courant **C** pour former un courant **D** comprenant trans-1-chloro-3,3,3-trifluoropropène ;
   caractérisé en ce que
l'étape iii) est mise en œuvre à une température **T2** comprise entre 50°C et 110°C, et l'écart de température, en valeur absolue, entre ladite température **T1** et ladite température **T2** est supérieur ou égal à 30°C.

Selon un mode de réalisation préféré, ledit réacteur comprend également des moyens de chauffage aptes à chauffer ladite phase liquide **A,** la température desdits moyens de chauffage est la température **T3** ; ladite température **T3** est supérieure à la température **T2** et ladite température **T3** est inférieure à 120°C.

Selon un mode de réalisation préféré, ladite phase liquide **A** fournie à l'étape i) est chauffée préalablement à la mise en œuvre de l'étape iii) à une température **T4** comprise entre 50°C et 110°C, de préférence la température **T4** est égale la température **T2.**

Selon un mode de réalisation préféré, ladite phase liquide **A** est une phase liquide pauvre en HF.

Selon un mode de réalisation préféré, ladite phase liquide **A** pauvre en HF est une phase liquide comprenant moins de 15% en poids de HF, avantageusement moins de 10% en poids de HF, de préférence moins de 8% en poids de HF, plus préférentiellement moins de 6% en poids de HF, en particulier moins de 5% en poids de HF, plus particulièrement moins de 4% en poids de HF, de manière privilégiée moins de 2% en poids de HF sur base du poids total de ladite phase liquide.

Selon un mode de réalisation préféré, la température **T2** est comprise entre 60°C et 105°C, de préférence entre 70°C et 100°C, plus préférentiellement entre 80°C et 100°C, en particulier entre 85°C et 95°C, plus particulièrement entre 88°C et 92°C.

Selon un mode de réalisation préféré, la température **T1** est comprise entre 120°C et 170°C, en particulier entre 125°C et 165°C, plus particulièrement entre 125°C et 155°C.

Selon un mode de réalisation préféré, la température **T3** est inférieure à 115°C, de préférence inférieure à 110°C, plus préférentiellement inférieure à 105°C, en particulier inférieure à 100°C.

Selon un mode de réalisation préféré, ledit courant **D** comprend également au moins un des produits secondaires sélectionnés parmi le groupe consistant en 1,1,1,3,3-pentafluoropropane, cis/trans-1,3,3,3-tétrafluoropropène et cis-1-chloro-3,3,3-trifluoropropène ; et la teneur molaire totale en ledit au moins un des produits secondaires est inférieure à 5 mol% dans ledit courant **D.**

Selon un mode de réalisation préféré, la teneur molaire en trans-1-chloro-3,3,3-trifluoropropène dans ledit courant **D** est supérieur à 95 mol%.

Selon un mode de réalisation préféré, l'étape iii) est mise en œuvre à une pression comprise entre 5 et 20 bara, de préférence entre 10 et 18 bara.

### Description détaillée de l'invention

La présente invention concerne un procédé de production du trans-1-chloro-3,3,3-trifluoropropène. En particulier, le présent procédé est mis en œuvre dans un réacteur comprenant un couvercle, un fond, des parois latérales connectant ledit fond et ledit couvercle, au moins une ligne d'alimentation en réactifs et au moins une ligne de soutirage des produits formés.

En outre, ledit réacteur contient une phase liquide **A.** Ladite phase liquide **A** est une phase liquide pauvre en HF ou une phase liquide riche en HF.

Ladite phase liquide **A** pauvre en HF est une phase liquide comprenant moins de 15% en poids de HF, avantageusement moins de 10% en poids de HF, de préférence moins de 8% en poids de HF, plus préférentiellement moins de 6% en poids de HF, en particulier moins de 5% en poids de HF, plus particulièrement moins de 4% en poids de HF, de manière privilégiée moins de 2% en poids de HF sur base du poids total de ladite phase liquide.

Ladite phase liquide **A** riche en HF est une phase liquide comprenant plus de 20% en poids de HF, avantageusement plus de 25% en poids de HF, de préférence plus de 30% en poids de HF, plus préférentiellement plus de 35% en poids de HF, en particulier plus de 40% en poids de HF, plus particulièrement plus de 45% en poids de HF, de manière privilégiée plus de 50% en poids de HF sur base du poids total de ladite phase liquide.

De préférence, ladite phase liquide **A** est une phase liquide pauvre en HF. En particulier, ladite phase liquide A comprend au moins 10% en poids de 1,1,3,3-tétrachloropropène et/ou 1,3,3,3-tétrachloropropène sur base du poids total de ladite composition de départ. Avantageusement, ladite composition de départ comprend au moins 15% en poids de 1,1,3,3-tétrachloropropène et/ou 1,3,3,3-tétrachloropropène, de préférence au moins 20% en poids de 1,1,3,3-tétrachloropropène et/ou 1,3,3,3-tétrachloropropène, plus préférentiellement au moins 25% en poids de 1,1,3,3-tétrachloropropène et/ou 1,3,3,3-tétrachloropropène, en particulier au moins 30% en poids de 1,1,3,3-tétrachloropropène et/ou 1,3,3,3-tétrachloropropène, plus particulièrement au moins 35% en poids de 1,1,3,3-tétrachloropropène et/ou 1,3,3,3-tétrachloropropène, de manière privilégiée au moins 40% en poids de 1,1,3,3-tétrachloropropène et/ou 1,3,3,3-tétrachloropropène, de manière avantageusement privilégiée au moins 45% en poids de 1,1,3,3-tétrachloropropène et/ou 1,3,3,3-tétrachloropropène, de manière préférentiellement privilégiée au moins 50% en poids de 1,1,3,3-tétrachloropropène et/ou 1,3,3,3-tétrachloropropène, de manière particulièrement privilégiée au moins 55% en poids de 1,1,3,3-tétrachloropropène et/ou 1,3,3,3-tétrachloropropène sur base du poids total de ladite phase liquide **A.**

De préférence, ladite phase liquide **A** comprend au moins 60% en poids ou au moins 65% en poids ou au moins 70% en poids ou au moins 75 % en poids ou au moins 80% en poids ou au moins 85% en poids ou au moins 90% en poids ou au moins 95% en poids ou au moins 99% en poids de 1,1,3,3-tétrachloropropène et/ou 1,3,3,3-tétrachloropropène sur base du poids total de ladite phase liquide **A.**

Ainsi, le présent procédé comprend l'étape i) de fourniture d'un réacteur comprenant un couvercle, un fond, des parois latérales connectant ledit fond et ledit couvercle, au moins une ligne d'alimentation en réactifs et au moins une ligne de soutirage des produits formés, ledit réacteur contenant en outre une phase liquide **A.**

Le courant B utilisé dans le présent procédé comprend de l'acide fluorhydrique. Le terme acide fluorhydrique utilisé ici englobe l'acide fluorhydrique et l'acide fluorhydrique anhydre. De préférence, l'acide fluorhydrique comprend moins de 1000 ppm d'eau, avantageusement moins de 800 ppm d'eau, de préférence moins de 600 ppm, plus préférentiellement moins de 400 ppm d'eau, en particulier moins de 200 ppm d'eau, plus particulièrement moins de 100 ppm d'eau, de manière privilégiée moins de 50 ppm d'eau.

De préférence, le courant **B** comprend au moins 50% en poids d'acide fluorhydrique, avantageusement au moins 60% en poids d'acide fluorhydrique, de préférence au moins 70% en poids d'acide fluorhydrique, plus préférentiellement au moins 80% en poids d'acide fluorhydrique, en particulier au moins 90% en poids d'acide fluorhydrique, plus particulièrement au moins 95% en poids d'acide fluorhydrique, de manière privilégiée au moins 99% en poids d'acide fluorhydrique sur base du poids total dudit courant **B.**

De préférence, le courant **B** est chauffé à une température **T1** comprise de 100°C à 170°C. Le courant **B** est chauffé avant son introduction dans ledit réacteur. Le chauffage dudit courant **B** peut être effectué par différents moyens tels qu'un traçage électrique, i.e. la ligne d'alimentation en réactifs contenant le courant **B** est enrobée d'une résistance électrique, un échangeur de chaleur ou une double-enveloppe disposée autour de la ligne d'alimentation en réactifs contenant le courant **B.** La double enveloppe contient un fluide caloporteur tel que par exemple de la vapeur, de l'eau chaude pressurisée ou de l'huile.

Le courant C utilisé dans le présent procédé comprend 1,1,3,3-tétrachloropropène et/ou 1,3,3,3-tétrachloropropène. De préférence, ledit courant au moins 50% en poids de 1,1,3,3-tétrachloropropène et/ou 1,3,3,3-tétrachloropropène, avantageusement au moins 60% en poids de 1,1,3,3-tétrachloropropène et/ou 1,3,3,3-tétrachloropropène, de préférence au moins 70% en poids de 1,1,3,3-tétrachloropropène et/ou 1,3,3,3-tétrachloropropène, plus préférentiellement au moins 80% en poids de 1,1,3,3-tétrachloropropène et/ou 1,3,3,3-tétrachloropropène, en particulier au moins 90% en poids de 1,1,3,3-tétrachloropropène et/ou 1,3,3,3-tétrachloropropène, plus particulièrement au moins 95% en poids de 1,1,3,3-tétrachloropropène et/ou 1,3,3,3-tétrachloropropène, de manière privilégiée au moins 99% en poids de 1,1,3,3-tétrachloropropène et/ou 1,3,3,3-tétrachloropropène sur base du poids total dudit courant **C.**

Ledit courant **B** et ledit courant **C** alimente ledit réacteur via une ou plusieurs lignes d'alimentation de réactifs. Ledit courant **B** ou ledit courant **C** peuvent éventuellement être mélangés avant d'être introduit dans ledit réacteur. Ledit courant **B** et/ou ledit courant **C** peuvent éventuellement être injectés dans ladite phase liquide **A** contenue dans ledit réacteur.

De préférence, lesdits courants **B** et **C** sont mis en contact en phase liquide. La réaction entre l'acide fluorhydrique et le 1,1,3,3-tétrachloropropène et/ou 1,3,3,3-tétrachloropropène permet la formation d'un courant **D** comprenant trans-1-chloro-3,3,3-trifluoropropène. De préférence, le courant **D** est un courant gazeux.

Ainsi, la présente invention fournit un procédé de production du trans-1-chloro-3,3,3-trifluoropropène comprenant les étapes de :
i) fourniture d'un réacteur comprenant un couvercle, un fond, des parois latérales connectant ledit fond et ledit couvercle, au moins une ligne d'alimentation en réactifs et au moins une ligne de soutirage des produits formés, ledit réacteur contenant en outre une phase liquide **A** ;
ii) fourniture d'un courant **B** comprenant de l'acide fluorhydrique chauffé à une température **T1** comprise de 100°C à 170°C et fourniture d'un courant **C** comprenant le 1,1,3,3-tétrachloropropène et/ou 1,3,3,3-tétrachloropropène ; ledit courant **B** et ledit courant **C** alimentant ledit réacteur par l'intermédiaire de ladite au moins une ligne d'alimentation en réactifs,
iii) réaction dans ladite phase liquide **A** dudit courant **B** avec ledit courant **C** pour former un courant **D** comprenant trans-1-chloro-3,3,3-trifluoropropène.

De préférence, l'étape iii) est mise en œuvre à une température **T2** comprise entre 50°C et 150°C, avantageusement entre 50°C et 140°C, de préférence entre 50°C et 130°C, plus préférentiellement entre 50°C et 120°C, en particulier entre 50°C et 110°C. Plus particulièrement l'étape iii) est mise en œuvre à une température **T2** comprise entre 60°C et 105°C, de préférence entre 70°C et 100°C, plus préférentiellement entre 80°C et 100°C, en particulier entre 85°C et 95°C, plus particulièrement entre 88°C et 92°C.

Selon un mode de réalisation préféré, l'écart de température, en valeur absolue, entre ladite température **T1** et ladite température **T2** est supérieur ou égal à 30°C. L'écart de température, en valeur absolue, entre ladite température **T1** et ladite température **T2** peut être supérieur ou égal à 32°C, ou supérieur ou égal à 34°C, ou supérieur ou égal à 36°C, ou supérieur ou égal à 38°C, ou supérieur ou égal à 40°C, ou supérieur ou égal à 42°C, ou supérieur ou égal à 44°C, ou supérieur ou égal à 46°C, ou supérieur ou égal à 48°C, ou supérieur ou égal à 50°C.

De préférence, l'écart de température, en valeur absolue, entre ladite température **T1** et ladite température **T2** est compris entre 30°C et 80°C, avantageusement entre 33°C et 75°C, de préférence entre 35°C et 70°C, en particulier entre 35°C et 65°C, plus particulièrement entre 35°C et 60°C.

Selon un mode de réalisation préféré, ledit réacteur comprend également un moyen de chauffage apte à chauffer la phase liquide **A.** Le moyen de chauffage peut chauffer les parois latérales du réacteur ou la phase liquide **A** directement. Par exemple, le moyen de chauffage peut être une double enveloppe disposée autour des parois latérales du réacteur, un serpentin disposé dans le réacteur et en contact avec ladite phase liquide A, une boucle de recirculation avec un échangeur de chaleur, celle-ci étant disposée à l'extérieur du réacteur. Le serpentin et la double enveloppe contiennent un fluide caloporteur tel que par exemple de la vapeur, de l'eau chaude pressurisée ou de l'huile. Lesdits moyens de chauffage permettent de fixer la température **T3.**

De préférence, ladite température **T3** est supérieure à la température **T2.** En outre, ladite température **T3** est inférieure à 120°C, avantageusement inférieure à 115°C, de préférence inférieure à 110°C, plus préférentiellement inférieure à 105°C, en particulier inférieure à 100°C.

Selon un mode de réalisation préféré, ladite phase liquide **A** fournie à l'étape i) est chauffée préalablement à la mise en œuvre de l'étape iii) à une température **T4** comprise entre 50°C et 150°C. De préférence, ladite phase liquide **A** est chauffée à une température **T4** comprise entre 50°C et 140°C, de préférence entre 50°C et 130°C, plus préférentiellement entre 50°C et 120°C, en particulier entre 50°C et 110°C. Plus particulièrement, ladite phase liquide **A** est chauffée à une température **T4** comprise entre 60°C et 105°C, de préférence entre 70°C et 100°C, plus préférentiellement entre 80°C et 100°C, en particulier entre 85°C et 95°C, plus particulièrement entre 88°C et 92°C. En particulier, ladite phase liquide **A** est chauffée à une température **T4** égale la température **T2.**

Selon un mode de réalisation préféré, la température **T1** est comprise entre 120°C et 170°C, avantageusement entre 125°C et 165°C, de préférence entre 125°C et 155°C.

Selon un mode de réalisation préféré, l'écart de température, en valeur absolue, entre ladite température **T3** et ladite température **T2** est inférieur ou égal à 30°C, en particulier inférieur ou égal à 25°C, plus particulièrement inférieur ou égal à 20°C.

De préférence, la température **T1** est comprise entre 120°C et 170°C, en particulier entre 125°C et 165°C, plus particulièrement entre 125°C et 155°C ; la température **T2** est comprise entre 80°C et 100°C, en particulier entre 85°C et 95°C, plus particulièrement entre 88°C et 92°C ; la température **T3** est supérieure à **T2** et inférieure à 120°C, avantageusement inférieure à 115°C, de préférence inférieure à 110°C, plus préférentiellement inférieure à 105°C, en particulier inférieure à 100°C ; et la température **T4** est comprise entre 80°C et 100°C, en particulier entre 85°C et 95°C, plus particulièrement entre 88°C et 92°C.

En particulier, la température **T1** est comprise entre 120°C et 170°C, en particulier entre 125°C et 165°C, plus particulièrement entre 125°C et 155°C ; la température **T2** est comprise entre 80°C et 100°C, en particulier entre 85°C et 95°C, plus particulièrement entre 88°C et 92°C ; la température **T3** est supérieure à **T2** et inférieure à 120°C, avantageusement inférieure à 115°C, de préférence inférieure à 110°C, plus préférentiellement inférieure à 105°C, en particulier inférieure à 100°C; la température **T4** est comprise entre 80°C et 100°C, en particulier entre 85°C et 95°C, plus particulièrement entre 88°C et 92°C ; et l'écart de température, en valeur absolue, entre ladite température **T3** et ladite température **T2** est inférieur ou égal à 30°C, en particulier inférieur ou égal à 25°C, plus particulièrement inférieur ou égal à 20°C.

Plus particulièrement, la température **T1** est comprise entre 120°C et 170°C, en particulier entre 125°C et 165°C, plus particulièrement entre 125°C et 155°C ; la température **T2** est comprise entre 80°C et 100°C, en particulier entre 85°C et 95°C, plus particulièrement entre 88°C et 92°C ; la température **T3** est supérieure à **T2** et inférieure à 120°C, avantageusement inférieure à 115°C, de préférence inférieure à 110°C, plus préférentiellement inférieure à 105°C, en particulier inférieure à 100°C ; la température **T4** est comprise entre 80°C et 100°C, en particulier entre 85°C et 95°C, plus particulièrement entre 88°C et 92°C ; l'écart de température, en valeur absolue, entre ladite température **T3** et ladite température **T2** est inférieur ou égal à 30°C, en particulier inférieur ou égal à 25°C, plus particulièrement inférieur ou égal à 20°C ; et l'écart de température, en valeur absolue, entre ladite température **T1** et ladite température **T2** est compris entre 30°C et 80°C, avantageusement entre 33°C et 75°C, de préférence entre 35°C et 70°C, en particulier entre 35°C et 65°C, plus particulièrement entre 35°C et 60°C.

De manière privilégiée, la température **T1** est comprise entre 120°C et 170°C, en particulier entre 125°C et 165°C, plus particulièrement entre 125°C et 155°C ; la température **T2** est comprise entre 80°C et 100°C, en particulier entre 85°C et 95°C, plus particulièrement entre 88°C et 92°C; la température **T3** est supérieure à **T2** et inférieure à 120°C, avantageusement inférieure à 115°C, de préférence inférieure à 110°C, plus préférentiellement inférieure à 105°C, en particulier inférieure à 100°C; la température **T4** est comprise entre 80°C et 100°C, en particulier entre 85°C et 95°C, plus particulièrement entre 88°C et 92°C ; l'écart de température, en valeur absolue, entre ladite température **T3** et ladite température **T2** est inférieur ou égal à 30°C, en particulier inférieur ou égal à 25°C, plus particulièrement inférieur ou égal à 20°C ; l'écart de température, en valeur absolue, entre ladite température **T1** et ladite température **T2** est compris entre 30°C et 80°C, avantageusement entre 33°C et 75°C, de préférence entre 35°C et 70°C, en particulier entre 35°C et 65°C, plus particulièrement entre 35°C et 60°C; et la température **T4** est égale à la température **T2.**

Selon un mode de réalisation préféré, ledit courant **D** comprend également au moins un des produits secondaires sélectionnés parmi le groupe consistant en 1,1,1,3,3-pentafluoropropane, cis/trans-1,3,3,3-tétrafluoropropène et cis-1-chloro-3,3,3-trifluoropropène. De préférence, la teneur molaire totale en 1,1,1,3,3-pentafluoropropane, cis/trans-1,3,3,3-tétrafluoropropène et cis-1-chloro-3,3,3-trifluoropropène est inférieure à 5 mol% dans ledit courant **D.** En particulier, la teneur molaire totale en 1,1,1,3,3-pentafluoropropane, cis/trans-1,3,3,3-tétrafluoropropène et cis-1-chloro-3,3,3-trifluoropropène est inférieure à 4,9 mol% dans ledit courant D. Plus particulièrement, la teneur molaire totale en 1,1,1,3,3-pentafluoropropane, cis/trans-1,3,3,3-tétrafluoropropène et cis-1-chloro-3,3,3-trifluoropropène est inférieure à 4,8 mol% dans ledit courant **D.** De manière privilégiée, la teneur molaire totale en 1,1,1,3,3-pentafluoropropane, cis/trans-1,3,3,3-tétrafluoropropène et cis-1-chloro-3,3,3-trifluoropropène est inférieure à 4,7 mol% dans ledit courant **D.** De manière avantageusement privilégiée, la teneur molaire totale en 1,1,1,3,3-pentafluoropropane, cis/trans-1,3,3,3-tétrafluoropropène et cis-1-chloro-3,3,3-trifluoropropène est inférieure à 4,6 mol% dans ledit courant **D.** De manière préférentiellement privilégiée, la teneur molaire totale en 1,1,1,3,3-pentafluoropropane, cis/trans-1,3,3,3-tétrafluoropropène et cis-1-chloro-3,3,3-trifluoropropène est inférieure à 4,5 mol% dans ledit courant **D.** De manière particulièrement privilégiée, la teneur molaire totale en 1,1,1,3,3-pentafluoropropane, cis/trans-1,3,3,3-tétrafluoropropène et cis-1-chloro-3,3,3-trifluoropropène est inférieure à 4,4 mol% dans ledit courant **D.**

La teneur molaire en trans-1-chloro-3,3,3-trifluoropropène dans ledit courant **D** peut être supérieur à 95 mol%. Avantageusement, la teneur molaire en trans-1-chloro-3,3,3-trifluoropropène dans ledit courant **D** peut être supérieur à 95,1 mol%. De préférence, la teneur molaire en trans-1-chloro-3,3,3-trifluoropropène dans ledit courant **D** peut être supérieur à 95,2 mol%. Plus préférentiellement, la teneur molaire en trans-1-chloro-3,3,3-trifluoropropène dans ledit courant **D** peut être supérieur à 95,3 mol%. En particulier, De préférence, la teneur molaire en trans-1-chloro-3,3,3-trifluoropropène dans ledit courant **D** peut être supérieur à 95,4 mol%. Plus particulièrement, la teneur molaire en trans-1-chloro-3,3,3-trifluoropropène dans ledit courant **D** peut être supérieur à 95,5 mol%. De manière privilégiée, la teneur molaire en trans-1-chloro-3,3,3-trifluoropropène dans ledit courant **D** peut être supérieur à 95,6 mol%. Les teneurs molaires sont exprimées sur base des composés organiques présents dans le courant considéré. Le courant **D** peut également comprendre HCl et HF. La teneur molaire mentionnée ci-dessus est celle obtenue en sortie du réacteur, c'est-à-dire avant purification.

De préférence, l'étape i) est mise en œuvre en l'absence de catalyseur.

Alternativement, l'étape i) peut être mise en œuvre en présence d'un catalyseur. Le catalyseur peut être un catalyseur de type TiCl₄ ou SbCl₅. Le catalyseur peut être également un liquide ionique. Les liquides ioniques pouvant convenir sont dérivés d'acides de Lewis à base d'aluminium, de titane, de niobium, de tantale, d'étain, d'antimoine, de nickel, de zinc ou de fer. On entend par liquides ioniques des sels non aqueux à caractère ionique qui sont liquides à des températures modérées (de préférence en-dessous de 120°C). Les liquides ioniques résultent de préférence de la réaction entre un sel organique et un composé inorganique. Les liquides ioniques sont de préférence obtenus par réaction d'au moins un acide de Lewis halogéné ou oxyhalogéné à base d'aluminium, de titane, de niobium, de tantale, d'étain, d'antimoine, de nickel, de zinc ou de fer avec un sel de formule générale Y⁺A⁻, dans laquelle A⁻ désigne un anion halogénure (bromure, iodure et, de préférence chlorure ou fluorure) ou hexafluoroantimoniate (SbF₆⁻) et Y⁺ un cation ammonium quaternaire, phosphonium quaternaire ou sulfonium ternaire. L'acide de Lewis halogéné à base d'aluminium, de titane, de niobium, de tantale, d'antimoine, de nickel, de zinc ou de fer peut être un dérivé chloré, bromé, fluoré ou mixte, par exemple un acide chlorofluoré. Peuvent être mentionnés plus particulièrement les chlorures, fluorures ou chlorofluorures des formules suivantes :
TiClₓF_{y} avec x+y = 4 et 0 <= x <= 4
TaClₓF_{y} avec x+y = 5 et 0 <= x <= 5
NbClₓF_{y} avec x+y = 5 et 0 <= x <= 5
SnClₓF_{y} avec x+y = 4 et 1 ≤ x ≤ 4
SbClₓF_{y} avec x+y = 5 et 0 <= x <= 5
AlClₓF_{y} avec x+y =3 et 0 <= x <= 3
NiClₓF_{y} avec x+y = 2 et 0 <= x <= 2
FeClₓF_{y} avec x+y = 3 et 0 <= x <= 3

Comme exemples de tels composés, on peut mentionner les composés suivants : TiCl₄, TiF₄, TaCl₅, TaF₅, NbCl₅, NbF₅, SbCl₅, SbCl₄F, SbCl₃F₂, SbCl₂F₃, SbClF₄, SbF₅ et leurs mélanges. Sont préferentiellement utilisés les composés suivants : TiCl₄, TaCl₅+TaF₅, NbCl₅+NbF₅, SbCl₅, SbFCl₄, SbF₂Cl₃, SbF₃Cl₂, SbF₄Cl, SbF₅ et SbCl₅+SbF₅. Sont plus particulièrement préférés les composés antimoniés. Comme exemples d'acides de Lewis oxyhalogénés, utilisables selon l'invention, on peut mentionner TiOCl₂, TiOF₂ et SbOClₓF_{y} (x+y=3). Dans le sel Y⁺A⁻, le cation Y⁺ peut répondre à l'une des formules générales suivantes: R¹R²R³R⁴N⁺, R¹R²R³R⁴P⁺, R¹R²R³S⁺ dans lesquelles les symboles R¹ a R⁴, identiques ou différents, désignent chacun un groupement hydrocarbyle, chlorohydrocarbyle, fluorohydrocarbyle, chlorofluorohydrocarbyle ou fluorocarbyle ayant de 1 a 10 atomes de carbone, saturé ou non, cyclique ou non, ou aromatique, l'un ou plusieurs de ces groupements pouvant également contenir un ou plusieurs hétéroatomes tels que N, P, S ou O. Le cation ammonium, phosphonium ou sulfonium Y⁺ peut également faire partie d'un hétérocycle saturé ou non, ou aromatique ayant de 1 à 3 atomes d'azote, de phosphore ou de soufre, et répondre à l'une ou l'autre des formules générales suivantes : 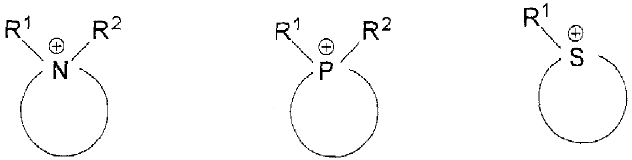 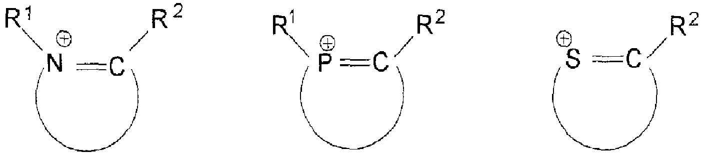 dans lesquelles R¹ et R² sont tels que définis précédemment. Un sel contenant 2 ou 3 sites ammonium, phosphonium ou sulfonium dans leur formule peut également convenir. Comme exemples de sels Y⁺A⁻ on peut mentionner les chlorures et fluorures de tétraalkyl ammonium, les chlorures et fluorures de tetraalkyl phosphonium, et chlorures et fluorures de trialkyl sulfonium, les chlorures et fluorures d'alkyl pyridinium, les chlorures, fluorures et bromures de dialkyl imidazolium, et les chlorures et fluorures de trialkyl imidazolium. Sont plus particulièrement appréciés le fluorure ou le chlorure de trimethyl sulfonium, le chlorure ou le fluorure de N-ethyl-pyridinium, le chlorure ou le fluorure de N-butyl-pyridinium, le chlorure ou le fluorure de 1-ethyl-3-methyl-imidazolium, et le chlorure ou fluorure de 1-butyl-3-methyl-imidazolium. Les liquides ioniques peuvent être préparés de façon connue en soi en mélangeant de manière appropriée l'acide de Lewis halogéné ou oxyhalogéné et le sel organique Y⁺A⁻. On peut se reporter notamment à la méthode décrite dans le document WO 01/81353. Alternativement, le catalyseur peut être l'acide triflique ou trifluoroacétique tel que mentionné dans le document US 6,166,274.

De préférence, l'étape iii) est mise en œuvre à une pression de 5 à 20 bara, de préférence à une pression de 10 à 18 bara, en particulier de 12 à 18 bara.

De préférence, le ratio molaire HF/[1,1,3,3-tétrachloropropène et/ou 1,3,3,3-tétrachloropropène] à l'entrée du réacteur est compris entre 5 et 10, plus préférentiellement entre 5 et 7, en particulier entre 5 et 6.

De préférence, ledit procédé comprend également les étapes de : (iv) au moins une étape de traitement du courant **D** pour donner un courant **E** comprenant le E-1-chloro-3,3,3-trifluoropropène, HCl, HF et le Z-1-chloro-3,3,3-trifluoropropène et un courant **F** comprenant majoritairement l'HF (par exemple au moins 50% en poids, de préférence au moins 70% en poids d'HF); (v) au moins une étape de récupération de l'acide chlorhydrique du courant **E** pour donner un courant **G** d'HCl et un courant **H** comprenant le E-1-chloro-3,3,3-trifluoropropène, HCl, HF et le Z-1-chloro-3,3,3-trifluoropropène ; (vi) au moins une étape de purification du courant **H** issu de l'étape (v) pour donner du E-1233zd, de préférence avec une pureté supérieure ou égale à 98 %, avantageusement supérieure ou égale à 99 %, et très avantageusement supérieure ou égale à 99,9 % en poids.

De préférence, avant l'étape de purification, le courant **H** issu de l'étape (v) est soumis à au moins une étape de séparation pour donner un flux comprenant principalement l'HF (par exemple au moins 90% en poids, de préférence au moins 98% en poids et avantageusement au moins 99% en poids d'HF) pouvant être recyclé au réacteur et un flux comprenant le E-1-chloro-3,3,3-trifluoropropène, HCl, HF et le Z-1-chloro-3,3,3-trifluoropropène. L'étape de séparation est de préférence une décantation, mise en œuvre à une température avantageusement comprise entre -50 et 50°C, de préférence entre -20°C et 10°C.

L'étape de traitement (iv) est de préférence une colonne de reflux, mise en œuvre avantageusement à une température comprise entre 30 et 120°C pour donner le courant F qui est recyclé au réacteur.

La récupération d'HCl à l'étape (v) est de préférence obtenue à l'aide d'une colonne de distillation munie d'un rebouilleur en pied et d'un système de reflux en tête. La température en pied est avantageusement comprise entre 20 et 110°C. La température en tête est avantageusement comprise entre -50 et 0°C. La distillation de l'HCl est typiquement effectuée à une pression comprise entre 7 et 25 bars.

Selon un mode de réalisation, l'étape de purification (vi) comprend de préférence au moins une étape de distillation et avantageusement au moins deux étapes de distillation. Selon un mode de réalisation préféré, l'étape de purification (vi) comprend au moins une étape de lavage à l'eau et/ou de lavage à l'aide d'une solution basique, une étape de séchage, et au moins une étape de distillation. Cette étape de distillation a pour but d'éliminer les produits légers et aussi les produits lourds qui peuvent être partiellement recyclés vers le réacteur, selon qu'ils sont recyclables ou non.

De préférence, le procédé est mis en œuvre en continu.

### Exemples

L'équipement utilisé consiste en un réacteur phase liquide d'une capacité de 60 litres utiles avec une double enveloppe, fabriqué en acier inoxydable 316L. Il est pourvu de moyens de mesure de température, de pression et de niveau liquide. Un tube plongeur permet d'alimenter les réactifs tandis que les produits formés circulent à travers une colonne de reflux de 5 mètres avant d'être condensés en tête. Cette colonne est remplie d'un garnissage métallique structuré qui permet de séparer les produits de faible point d'ébullition tandis que la matière première, les composés intermédiaires et le HF non réagi sont rétrogradés dans le réacteur. Une vanne de régulation de pression impose une pression de fonctionnement à l'ensemble. Un système de prélèvement en ligne permet d'échantillonner le flux de gaz de sortie qui est ainsi orienté vers un appareil de chromatographie gazeuse. Les réactifs sont alimentés en continu et les produits sont analysés et collectés en continu.

### Exemple 1 (selon l'invention)

Une quantité de 25 litres d'une phase liquide **A** comprenant 1,1,3,3-tétrachloropropène est introduite dans un réacteur. L'alimentation en HF est préchauffée à l'aide d'une double enveloppe alimentée par de la vapeur surchauffée. La température **T1** est de 130°C. La phase liquide **A** est préchauffée à 90°C à l'aide d'un traçage électrique, soit une température **T4** de 90°C. La double enveloppe du réacteur est ensuite alimentée en eau chaude à l'aide d'une chaudière. La température **T3** des parois est de 95°C. La température **T2** de réaction, dans ce cas la température de la phase liquide pendant la réaction de fluoration, est de 90°C. La régulation de pression est ajustée à 15 bara. Le ratio molaire entre l'HF et 1,1,3,3-tétrachloropropène est de 6. Les résultats de composition du flux gazeux sont donnés dans le tableau 1.

### Exemple 2 (selon l'invention)

La procédure de l'exemple 1 est reproduite avec une température de HF entrant dans le réacteur de 123°C (**T1**). La température **T3** est de 97°C. La température **T2** et la température **T4** sont de 90°C. Le réacteur est chauffé de la même manière que précédemment. Le ratio molaire entre l'HF et 1,1,3,3-tétrachloropropène est de 6. Les résultats de composition du flux gazeux sont donnés dans le tableau 1.

### Exemple 3 (Comparatif)

La procédure de l'exemple 1 est reproduite une température de HF entrant dans le réacteur de 117°C (**T1**). La température **T3** est de 100°C. La température **T2** et la température **T4** sont de 90°C. Le réacteur est chauffé de la même manière que précédemment. Le ratio molaire entre l'HF et 1,1,3,3-tétrachloropropène est de 6. Les résultats de composition du flux gazeux sont donnés dans le tableau 1.

**Tableau 1**

| | F1233zd-E (mol%) | F1233zdZ + F1234ze + 245fa (mol%) |
|---|---|---|
| Exemple 1 (Inv.) | 95,4 | 4,31 |
| Exemple 2 (Inv.) | 95,4 | 4,33 |
| Exemple 3 (Comp.) | 94,4 | 5,10 |

Les valeurs mentionnées représentent la teneur molaire des constituants cités dans le courant gazeux obtenu en sortie du réacteur, la teneur est exprimée sur base des composés organiques présents dans le réacteur, c'est-à-dire sans tenir compte de la teneur en HCl et en HF dans ce courant.

Les résultats détaillés dans le tableau 1 démontrent que le procédé selon la présente invention génère moins de sous-produits de réaction et de produits surfluorés. Le chauffage de l'acide fluorhydrique combiné à un contrôle de la température des parois du réacteur permet d'aboutir à un procédé de production du trans-1-chloro-3,3,3-trifluoropropène plus efficace.

## Revendications

1. Procédé de production du trans-1-chloro-3,3,3-trifluoropropène comprenant les étapes de :
i) fourniture d'un réacteur comprenant un couvercle, un fond, des parois latérales connectant ledit fond et ledit couvercle, au moins une ligne d'alimentation en réactifs et au moins une ligne de soutirage des produits formés, ledit réacteur contenant en outre une phase liquide A ;
ii) fourniture d'un courant B comprenant de l'acide fluorhydrique chauffé à une température **T1** comprise de 100°C à 170°C et fourniture d'un courant C comprenant le 1,1,3,3-tétrachloropropène et/ou 1,3,3,3-tétrachloropropène ; ledit courant B et ledit courant C alimentant ledit réacteur par l'intermédiaire de ladite au moins une ligne d'alimentation en réactifs,
iii) réaction dans ladite phase liquide **A** dudit courant **B** avec ledit courant **C** pour former un courant **D** comprenant trans-1-chloro-3,3,3-trifluoropropène ;
**caractérisé en ce que**
l'étape iii) est mise en œuvre à une température **T2** comprise entre 50°C et 110°C, et
l'écart de température, en valeur absolue, entre ladite température **T1** et ladite température **T2** est supérieur ou égal à 30°C.

2. Procédé selon la revendication 1 **caractérisé en ce que** ledit réacteur comprend également des moyens de chauffage aptes à chauffer ladite phase liquide **A,** la température desdits moyens de chauffage est la température **T3** ; ladite température **T3** est supérieure à la température **T2** et ladite température **T3** est inférieure à 120°C.

3. Procédé selon l'une quelconque des revendications précédentes **caractérisé en ce que** ladite phase liquide **A** fournie à l'étape i) est chauffée préalablement à la mise en œuvre de l'étape iii) à une température **T4** comprise entre 50°C et 110°C, de préférence la température **T4** est égale la température **T2.**

4. Procédé selon l'une quelconque des revendications précédentes **caractérisé en ce que** ladite phase liquide **A** est une phase liquide pauvre en HF.

5. Procédé selon la revendication précédente **caractérisé en ce que** ladite phase liquide A pauvre en HF est une phase liquide comprenant moins de 15% en poids de HF, avantageusement moins de 10% en poids de HF, de préférence moins de 8% en poids de HF, plus préférentiellement moins de 6% en poids de HF, en particulier moins de 5% en poids de HF, plus particulièrement moins de 4% en poids de HF, de manière privilégiée moins de 2% en poids de HF sur base du poids total de ladite phase liquide.

6. Procédé selon l'une quelconque des revendications précédentes **caractérisé en ce que** la température **T2** est comprise entre 60°C et 105°C, de préférence entre 70°C et 100°C, plus préférentiellement entre 80°C et 100°C, en particulier entre 85°C et 95°C, plus particulièrement entre 88°C et 92°C.

7. Procédé selon l'une quelconque des revendications précédentes **caractérisé en ce que** la température **T1** est comprise entre 120°C et 170°C, en particulier entre 125°C et 165°C, plus particulièrement entre 125°C et 155°C.

8. Procédé selon l'une quelconque des revendications précédentes 2 à 7 **caractérisé en ce que** la température **T3** est inférieure à 115°C, de préférence inférieure à 110°C, plus préférentiellement inférieure à 105°C, en particulier inférieure à 100°C.

9. Procédé selon l'une quelconque des revendications précédentes **caractérisé en ce que** ledit courant **D** comprend également au moins un des produits secondaires sélectionnés parmi le groupe consistant en 1,1,1,3,3-pentafluoropropane, cis/trans-1,3,3,3-tétrafluoropropène et cis-1-chloro-3,3,3-trifluoropropène ; et la teneur molaire totale en ledit au moins un des produits secondaires est inférieure à 5 mol% dans ledit courant **D.**

10. Procédé selon l'une quelconque des revendications précédentes **caractérisé en ce que** la teneur molaire en trans-1-chloro-3,3,3-trifluoropropène dans ledit courant **D** est supérieur à 95 mol%.

11. Procédé selon l'une quelconque des revendications précédentes **caractérisé en ce que** l'étape iii) est mise en œuvre à une pression comprise entre 5 et 20 bara, de préférence entre 10 et 18 bara.

## Patentansprüche

1. Verfahren zur Herstellung von trans-1-Chlor-3,3,3-trifluorpropen, das folgende Schritte umfasst:
i) Bereitstellen eines Reaktors mit einer Abdeckung, einem Boden, Seitenwänden, die den Boden und die Abdeckung verbinden, mindestens einer Leitung zum Einspeisen von Reagenzien und mindestens einer Leitung zum Abziehen der gebildeten Produkte, wobei der Reaktor außerdem eine flüssige Phase **A** enthält;
ii) Bereitstellen eines Fluorwasserstoffsäure umfassenden, auf eine Temperatur **T1** von 100 °C bis 170 °C erhitzten Stroms **B** und Bereitstellen eines 1,1,3,3-Tetrachlorpropen und/oder 1,3,3,3-Tetrachlorpropen umfassenden Stroms **C;** wobei der Strom **B** und der Strom **C** den Reaktor über die mindestens eine Leitung zum Einspeisen von Reagenzien speisen,
iii) Umsetzen des Stroms **B** mit dem Strom **C** in der flüssigen Phase **A** zur Bildung eines trans-1-Chlor-3,3,3-trifluorpropen umfassenden Stroms **D;**
**dadurch gekennzeichnet, dass**
der Schritt iii) bei einer Temperatur **T2** zwischen 50 °C und 110 °C durchgeführt wird und
die Temperaturdifferenz zwischen der Temperatur **T1** und der Temperatur **T2** als Absolutwert größer als oder gleich 30 °C ist.

2. Verfahren nach Anspruch 1, **dadurch gekennzeichnet, dass** der Reaktor außerdem Erhitzungsmittel zum Erhitzen der flüssigen Phase **A** umfasst, die Temperatur der Erhitzungsmittel die Temperatur **T3** ist; die Temperatur **T3** höher als die Temperatur **T2** ist und die Temperatur **T3** kleiner als 120 °C ist.

3. Verfahren nach einem der vorhergehenden Ansprüche, **dadurch gekennzeichnet, dass** die in Schritt i) bereitgestellte flüssige Phase **A** vor der Durchführung von Schritt iii) auf eine Temperatur **T4** zwischen 50 °C und 110 °C erhitzt wird, wobei die Temperatur **T4** vorzugsweise gleich der Temperatur **T2** ist.

4. Verfahren nach einem der vorhergehenden Ansprüche, **dadurch gekennzeichnet, dass** es sich bei der flüssigen Phase **A** um eine HF-arme flüssige Phase handelt.

5. Verfahren nach dem vorhergehenden Anspruch, **dadurch gekennzeichnet, dass** es sich bei der HF-armen flüssigen Phase **A** um eine flüssige Phase handelt, die weniger als 15 Gew.-% HF, vorteilhafterweise weniger als 10 Gew.-% HF, vorzugsweise weniger als 8 Gew.-% HF, weiter bevorzugt weniger als 6 Gew.-% HF, insbesondere weniger als 5 Gew.-% HF, spezieller weniger als 4 Gew.-% HF, bevorzugt weniger als 2 Gew.-% HF, bezogen auf das Gesamtgewicht der flüssigen Phase, umfasst.

6. Verfahren nach einem der vorhergehenden Ansprüche, **dadurch gekennzeichnet, dass** die Temperatur **T2** zwischen 60 °C und 105 °C, vorzugsweise zwischen 70 °C und 100 °C, weiter bevorzugt zwischen 80 °C und 100 °C, insbesondere zwischen 85 °C und 95 °C, spezieller zwischen 88 °C und 92 °C, liegt.

7. Verfahren nach einem der vorhergehenden Ansprüche, **dadurch gekennzeichnet, dass** die Temperatur **T1** zwischen 120 °C und 170 °C, insbesondere zwischen 125 °C und 165 °C, weiter bevorzugt zwischen 125 °C und 155 °C, liegt.

8. Verfahren nach einem der vorhergehenden Ansprüche 2 bis 7, **dadurch gekennzeichnet, dass** die Temperatur **T3** kleiner als 115 °C, vorzugsweise kleiner als 110 °C, weiter bevorzugt kleiner als 105 °C, insbesondere kleiner als 100 °C, ist.

9. Verfahren nach einem der vorhergehenden Ansprüche, **dadurch gekennzeichnet, dass** der Strom **D** außerdem mindestens eines der Nebenprodukte aus der Gruppe bestehend aus 1,1,1,3,3-Pentafluorpropan, cis/trans-1,3,3,3-Tetrafluorpropen und cis-1-Chlor-3,3,3-tri-fluorpropen umfasst und der gesamte molare Gehalt an dem mindestens einen der Nebenprodukte in dem Strom D kleiner als 5 Mol-% ist.

10. Verfahren nach einem der vorhergehenden Ansprüche, **dadurch gekennzeichnet, dass** der molare Gehalt an trans-1-Chlor-3,3,3-trifluorpropen in dem Strom D größer als 95 Mol-% ist.

11. Verfahren nach einem der vorhergehenden Ansprüche, **dadurch gekennzeichnet, dass** der Schritt iii) bei einem Druck zwischen 5 und 20 bara, vorzugsweise zwischen 10 und 18 bara, durchgeführt wird.

## Claims

1. Process for the production of trans-1-chloro-3,3,3-trifluoropropene, comprising the steps of:
i) providing a reactor comprising a cover, a bottom, sidewalls connecting said bottom and said cover, at least one reagent supply line and at least one line for drawing off the products formed, said reactor further containing a liquid phase **A;**
ii) providing a stream **B** comprising hydrofluoric acid heated to a temperature **T1** of from 100°C to 170°C and providing a stream **C** comprising 1,1,3,3-tetrachloropropene and/or 1,3,3,3-tetrachloropropene; said stream **B** and said stream **C** supplying said reactor via said at least one reagent supply line;
iii) reacting, in said liquid phase **A,** said stream **B** with said stream **C** in order to form a stream **D** comprising trans-1-chloro-3,3,3-trifluoropropene;
**characterized in that**
step iii) is carried out at a temperature **T2** of between 50°C and 110°C, and
the temperature difference, in absolute value, between said temperature **T1** and said temperature **T2** is greater than or equal to 30°C.

2. Process according to Claim 1, **characterized in that** said reactor also comprises heating means capable of heating said liquid phase **A;** the temperature of said heating means is the temperature **T3;** said temperature **T3** is higher than the temperature **T2** and said temperature **T3** is less than 120°C.

3. Process according to either one of the preceding claims, **characterized in that** said liquid phase **A** provided in step i) is heated prior to the implementation of step iii) to a temperature **T4** of between 50°C and 110°C; preferably, the temperature **T4** is equal to the temperature **T2.**

4. Process according to any one of the preceding claims, **characterized in that** said liquid phase **A** is a liquid phase low in HF.

5. Process according to the preceding claim, **characterized in that** said liquid phase **A** low in HF is a liquid phase comprising less than 15% by weight of HF, advantageously less than 10% by weight of HF, preferably less than 8% by weight of HF, more preferentially less than 6% by weight of HF, in particular less than 5% by weight of HF, more particularly less than 4% by weight of HF, preferably less than 2% by weight of HF, based on the total weight of said liquid phase.

6. Process according to any one of the preceding claims, **characterized in that** the temperature **T2** is between 60°C and 105°C, preferably between 70°C and 100°C, more preferentially between 80°C and 100°C, in particular between 85°C and 95°C, more particularly between 88°C and 92°C.

7. Process according to any one of the preceding claims, **characterized in that** the temperature **T1** is between 120°C and 170°C, in particular between 125°C and 165°C, more particularly between 125°C and 155°C.

8. Process according to any one of the preceding Claims 2 to 7, **characterized in that** the temperature **T3** is less than 115°C, preferably less than 110°C, more preferentially less than 105°C, in particular less than 100°C.

9. Process according to any one of the preceding claims, **characterized in that** said stream **D** also comprises at least one of the by-products selected from the group consisting of 1,1,1,3,3-pentafluoropropane, cis/trans-1,3,3,3-tetrafluoropropene and cis-1-chloro-3,3,3-trifluoropropene; and the total molar content of said at least one of the by-products is less than 5 mol% in said stream **D.**

10. Process according to any one of the preceding claims, **characterized in that** the molar content of trans-1-chloro-3,3,3-trifluoropropene in said stream **D** is greater than 95 mol%.

11. Process according to any one of the preceding claims, **characterized in that** step iii) is carried out at a pressure of between 5 and 20 bara, preferably between 10 and 18 bara.
